# EUROPÄISCHE PATENTSCHRIFT

(11) **EP 1 857 539 B1**
(45) Veröffentlichungstag und Bekanntmachung des Hinweises auf die Patenterteilung: **21.04.2010**
(21) Anmeldenummer: 07016403.3
(22) Anmeldetag: 11.01.2003
(51) Int. Cl.: C12M 3/00

(54) **Vorrichtung zum Züchten oder Kultivieren von Zellen in einem dosenartigen Behälter**
Device for raising or cultivating cells in a container-like receptacle
Dispositif de culture ou de mise en culture de cellules dans un récipient de type boite

(30) Priorität: 15.01.2002 DE 10201259
(43) Veröffentlichungstag der Anmeldung: 21.11.2007
(62) Teilanmeldung aus: 03704378.3
(73) Patentinhaber: Bader, Augustinus, 04668 Parthenstein / OT Klinga (DE)
(72) Erfinder: Bader, Augustinus, 04668 Parthenstein / OT Klinga (DE)
(74) Vertreter: Benz, Jürgen

(56) Entgegenhaltungen:
- WO-A-02/24861
- DE-A- 19 631 997
- DE-A- 19 710 650
- US-A- 5 219 755
- US-A- 5 267 791
- PATENT ABSTRACTS OF JAPAN Bd. 2000, Nr. 06, 22. September 2000 (2000-09-22) & JP 2000 078963 A (KOIZUMI YOSHIO), 21. März 2000 (2000-03-21)
- PATENT ABSTRACTS OF JAPAN Bd. 1996, Nr. 10, 31. Oktober 1996 (1996-10-31) -& JP 08 154663 A (ABLE KK), 18. Juni 1996 (1996-06-18)

## Beschreibung

Die Erfindung betrifft eine Vorrichtung zum Züchten oder Kultivieren von Zellen in einem dosenartigen Behälter, der einem oberen Deckel (3) und einem unteren als Boden dienender Deckel (12) sowie einem zylinderförmigen Mittelteil (1) aufweist, wobei Deckel und Mittelteil mit einem Gewindeverschluss verbunden sind. Die Erfindung betrifft insbesondere eine entsprechende Vorrichtung, welche Einrichtungen zur inneren Druckbeaufschlagung von Zellen aufweist.

Für den Laborbedarf ist es bekannt, Zellkulturen in einem flachen Behälter bzw. einer Schale zu züchten oder zu kultivieren, wobei die Zellen einfach in den Behälter eingelegt und Nährmedium zugegeben wird. Anschließend wird auf den Behälter ein Deckel aufgelegt.

Nachteilig dabei ist, dass dieses Verfahren nur für geringe Mengen angewendet werden kann. Insbesondere für eine Serienkultivierung oder Serienzüchtung von Zellen ist das bekannte System nicht geeignet. Darüber hinaus können keine "in-vivo"-Verhältnisse erreicht werden und es ist keine Sterilität gesichert.

Eine Alternative dazu bestand darin, dass mit einem geschlossenen System gearbeitet wurde, das einen Behälter mit einem Deckel oder Verschluss aufwies, wobei eine Sterilität möglich war. Nachteilig dabei war jedoch, dass die Entnahme der Zellkultur sehr umständlich und aufwendig war.

Der vorliegenden Erfindung liegt daher die Aufgabe zugrunde, eine Vorrichtung der eingangs erwähnte Art derart zu verbessern, dass sie auf die vielfältigste Weise zum Züchten oder Kultivieren von Zellen verwendet werden kann, insbesondere im Massenbetrieb, wobei soweit wie möglich "in-vivo"-Verhältnisse und eine Sterilität erreicht werden sollen. Außerdem sollen sich die Zellkulturen nach ihrer Züchtung auch ohne deren Beschädigung ohne großen Aufwand aus dem Behälter entnehmen lassen.

Erfindungsgemäß wird diese Aufgabe durch die im kennzeichnenden Teil von Anspruch 1 genannten Merkmale gelöst.

Entsprechend der erfindungsgemäßen Vorrichtung werden die Zellen in dem Behälter nunmehr "nicht mehr nur sich selbst überlassen", sondern es findet praktisch ein aktiver Prozess statt. Zum einen kann kontinuierlich oder diskontinuierlich Nährmedium zugeführt werden und zum anderen ist es möglich, die sich bildenden Zellkulturen mit Druck zu beaufschlagen. Dabei kann die Druckbeaufschlagung sogar mit wechselndem Druck erfolgen, um natürliche Verhältnisse so weit wie möglich nachzubilden.

Die Zellen können dabei als separate Zellkulturen gezüchtet oder kultiviert werden. In gleicher Weise können sie auch auf Strukturen gebildet werden, um Implantate zu erzeugen.

In dem erfindungsgemäßen Behälter können auf diese Weise durch eine entsprechende Druckbeaufschlagung die Zellen auch Scher- oder Druckkräften ausgesetzt werden. Mit der erfindungsgemäßen Vorrichtung lassen sich die verschiedensten Zellkulturen auf sehr universelle Weise züchten oder kultivieren.

Der Behälter selbst ist für diesen Zweck mit zwei Deckeln versehen. Ebenso ist eine Anschlussbohrung für den Zulauf von Nährmedium und für dessen Rücklauf vorgesehen sein. Selbstverständlich sind auch getrennte Anschlussbohrungen möglich, wobei Parallelströmungen oder auch Durchströmungen, je nach Anordnung der Anschlussbohrungen, möglich sind.

Durch die erfindungsgemäße Ausgestaltung mit zwei Deckeln oder Deckel und Boden nebst dazwischenliegendem Behälter, der in einfacher Weise lediglich ein auf der Unterseite und der Oberseite offener Zylinder sein kann, können die Zellkulturen nach ihrer Behandlung bzw. Züchtung ohne großen Aufwand und ohne deren Schädigung auf einfache Weise aus dem Behälter entnommen werden.

Da erfindungsgemäß die Züchtung oder Kultivierung der Zellen auf dem unteren Deckel oder Boden oder auch unter dem oberen Deckel, z.B. auf einem mit dem oberen Deckel verbundenen Gestell erfolgt, können die Zellkulturen nach ihrer Fertigstellung auf einfache Weise aus dem Behälter entnommen werden. Auf jeden Fall ist durch die abnehmbaren Deckel oder .Boden eine hohe Zugänglichkeit gegeben.

Für eine Druckbeaufschlagung kann der Behälter, z.B. die zylinderförmige Behälterumfangswand, auch elastisch ausgebildet sein.

Die Anschlussbohrung oder die Anschlussbohrungen können dabei in beiden Deckeln angeordnet sein. Ebenso ist es auch möglich, die Anschlussbohrungen in den zylinderförmigen Mittelteil einzubringen. Die Anzahl und die Anordnung der Anschlussbohrungen richtet sich dabei jeweils nach dem Einsatzfall und der zu züchtenden oder kultivierenden Zellen.

Für eine dichte Verbindung zwischen den Deckeln und dem Behälter sind Gewindeverbindungen mit Innen- und Außengewinden vorgesehen.

Sehr gute Abdicht- und damit Druckverhältnisse ergeben sich, wenn der oder die Deckel mit Verlängerungsringen versehen sind, die dann außenseitig dichtend den zylinderförmigen Mittelteil des Behälters umschließen.

Wenn man die erfindungsgemäße Vorrichtung einer Roll- oder Drehbewegung unterwerfen möchte, können seitlich auf die Vorrichtung Spannringe aufgebracht werden, die den oder die Deckel und den Behälter umfassen und an denen dann Dreh- oder Rolleinrichtungen angebracht werden können.

Für ein Unterdrucksetzen des einen Zellkulturraum bildenden Inneren des Behälters sind die verschiedenartigsten Druckbeaufschlagungseinrichtungen möglich. Hierzu sind z.B. Zylinder-/Kolbeneinheiten geeignet, die auch für eine wechselnde Druckbelastung entsprechend pulsierend operieren können.

Im Bedarfsfalle kann man den Behälter auch als Zweikammersystem ausbilden, um zwei verschiedene Zellen oder auch zwei gleiche Zellen getrennt voneinander kultivieren oder züchten zu können.

In diesem Falle ist es von Vorteil, wenn man den Deckel des Behälters mit einer Hängeeinrichtung versieht, an der eine Plattform zur Aufnahme von Zellen angeordnet ist. Damit erfolgt die Züchtung von einer Zellenart auf der Plattform, während eine weitere Zellkultivierung auf dem Boden des Behälters erfolgen kann.

Der Behälter bzw. die zylindrische Umfangswand des Behälters kann im Bedarfsfalle ebenfalls porös bzw. gasdurchlässig ausgebildet sein, so dass auf diese Weise ebenfalls noch Nährmittel und/oder ein gasförmiges Medium, wie z.B. Luft oder Sauerstoff, von dieser Seite aus zugeführt werden kann.

Vorteilhafte Weiterbildungen und Ausgestaltungen ergeben sich aus den übrigen Unteransprüchen und aus den nachfolgend anhand der Zeichnung prinzipmäßig dargestellten Ausführungsbeispielen.

Es zeigt:
Fig. 1 einen Schnitt durch eine Vorrichtung mit einem zylinderförmigen Mittelteil und einem oberen und einem unteren Deckel;
Fig. 2 eine ähnliche Ausgestaltung wie in der Fig. 2 dargestellt, wobei ein Zulaufanschluss und ein Rücklaufanschluss im oberen Deckel angeordnet sind;
Fig. 3 eine weitere Ausführungsform der erfindungsgemäßen Vorrichtungsform;
Fig. 4 eine erfindungsgemäße Vorrichtung mit einem oberen Deckel und einem unteren Deckel, jeweils mit Außengewinde und einem zylinderringförmigen Mittelteil mit Innengewinde;
Fig. 5 eine Ausführungsform mit Verlängerungsringen an einem oberen Deckel und einem unteren Deckel;
Fig. 6 eine ähnliche Ausgestaltung wie in Fig. 6 und 7 mit Gewindeverbindungen;
Fig. 7 eine Ausführungsform mit zwei seitlichen Spannringen;
Fig. 8 eine Ausführungsform ähnlich der Ausführungsform nach Fig. 7 mit einer Druckbeaufschlagungseinrichtung;
Fig. 9 eine Ausführungsform mit einer Hängeeinrichtung im oberen Deckel, mit einem zylinderförmigen Mittelteil und einem unteren Deckel;
Fig. 10 eine ähnliche Ausgestaltung wie Fig. 9 in etwas einfacher Form mit nur einem oberen Deckel;
Fig. 11 eine Ausgestaltung mit einer durch Magnetkräften erzeugten Druckkraft;
Fig. 11a, 11b und 11c verschiedene Druckscheibenprofile; Fig. 11d eine mineralische Matrix für Knochenersatz als Trägerstruktur mit zwei Druckscheiben;
Fig. 12 eine Ausgestaltung mit expansionsfähigen Elementen zur Erzeugung von Druckkräften;
Fig. 13 eine Ausgestaltung mit hydraulischen oder pneumatischen Elementen zur Erzeugung von Druckkräften;
Fig. 14 eine ähnliche Ausgestaltung wie die in der Fig. 13 besprochene Ausgestaltung;

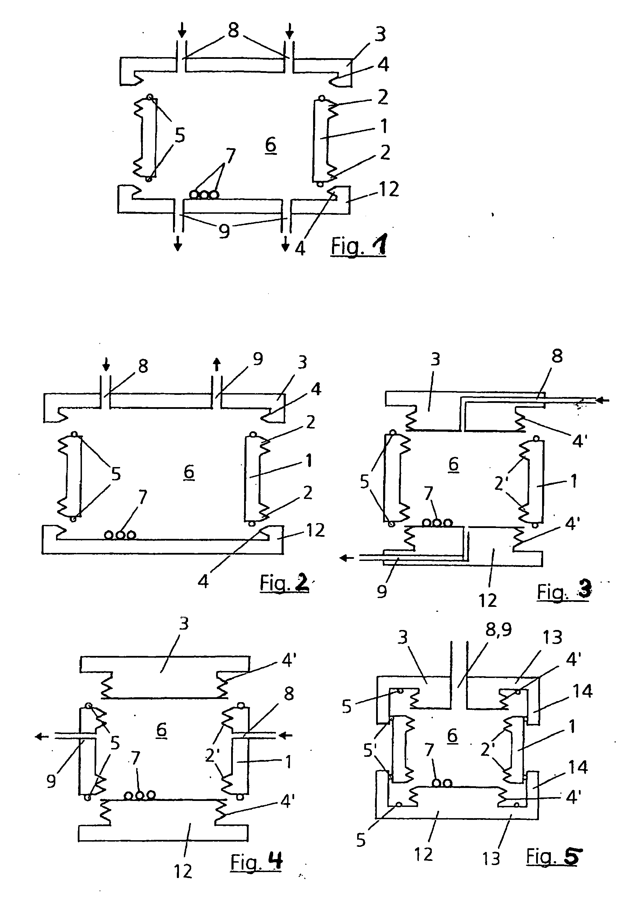

Fig. 1 zeigt eine Ausgestaltung, wobei der Behälter ein zylinderförmiges Mittelteil (1) bildet, der mit dem oberen Deckel 3 abgeschlossen werden kann. Auf der Unterseite ist ein weiterer unterer Deckel 12 vorgesehen, der den Boden des Behälters bildet und der ebenfalls ein Innengewinde 4 aufweist, welches mit dem Außengewinde 2 des Mittelteiles zusammenwirkt, wobei in diesem Fall das Mittelteil mit zwei Außengewinden 2 versehen ist, sofern nicht ein durchgehendes Gewinde vorgesehen ist. Wie weiterhin aus der Fig. 1 ersichtlich ist, sind dabei Zu- und Rücklaufanschlussbohrungen 8 und 9 in beiden Deckeln 3 und 12 vorgesehen.

Fig. 2 zeigt eine ähnliche Ausgestaltung wie Fig. 1, wobei eine Zulaufanschlussbohrung 8 und ein Rücklaufanschlussbohrung 9 nur im oberen Deckel 3 angeordnet sind. Ebenso wie bei dem Ausführungsbeispiel nach Fig. 2 sind an beiden Stirnseiten des zylinderringförmigen Mittelteiles (1) des Behälters Dichtringe 5 vorgesehen.

Fig. 3 zeigt eine Ausführungsform, wobei das zylinderförmige Mittelteil als Behälter mit einem Innengewinde 2' versehen ist, welches jeweils mit einem Außengewinde 4' des oberen Deckels 3 und des unteren Deckels 12 zusammenarbeitet. Auch hier sind wiederum auf beiden Stirnseiten des Behälters Dichtringe 5 vorgesehen. In diesem Fall befindet sich eine Zulaufanschlussbohrung 8 im oberen Deckel 3 und eine Rücklaufanschlussbohrung 9 im unteren Deckel 12.

Fig. 4 zeigt eine ähnliche Ausgestaltung wie Fig. 3. Der wesentliche Unterschied besteht lediglich darin, dass die Zulaufanschlussbohrung 8 und die Rücklaufanschlussbohrung 9 sich gegenüberliegend in dem zylinderringförmigen Mittelteil (1) des Behälters angeordnet sind.

Fig. 5 zeig eine Ausführungsform mit einem zylinderringförmigen Mittelteil (1) als Behälter, einem oberen Deckel 3 und einem unteren Deckel 12. Dabei weisen beide Deckel 3 und 12 jeweils ein Außengewinde 4' auf, welches mit Innengewinden 2' des Behälters zusammenwirkt. Zusätzlich sind beide Deckel 3 und 12 mit radialen Erweiterungen 13 versehen, von deren äußeren Enden aus sich parallel zur Längsachse des Behälters zu dem Behälter gerichtete bzw. axial gerichtete Verlängerungsringe 14 erstrecken. Die Verlängerungsringe 14 umschließen die Außenwand des Behälters und stellen zusammen mit zusätzlichen Dichtringen 5' einen druckdichten Abschluss für den Zellkulturraum 6 dar. Der obere Deckel 3 ist mit einer gemeinsamen Anschlussbohrung 8, 9 für die Zufuhr und den Rücklauf von Nährmedium versehen.

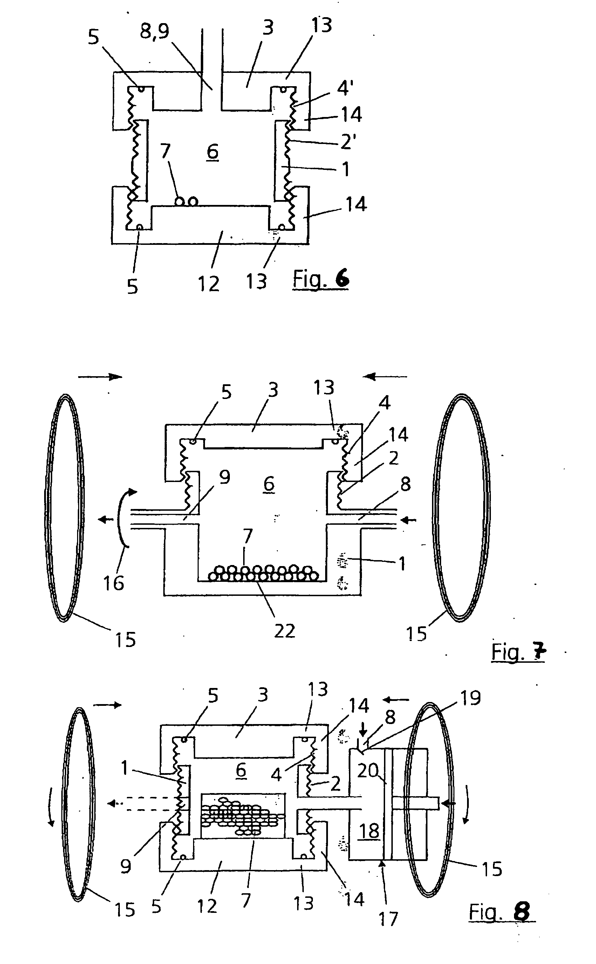

Fig. 6 zeigt eine Ausführungsform, ähnlich denen nach den Fig. 5. Der wesentliche Unterschied besteht dabei darin, dass die Gewindeverbindung zwischen dem Behälter und den Deckeln 3 und 12 durch Innengewinde 4' in den Verlängerungsringen 14 gebildet ist, die mit Außengewinden 2' in dem Behälter zusammenwirken.

Fig. 7 zeigt eine Ausführungsform mit einem Behälter und einem oberen Deckel 3, ähnlich der Ausführungsform nach der Fig. 6, wobei anstelle einer gemeinsamen Anschlussbohrung 8, 9 für die Zufuhr und die Abfuhr von Nährmedium in der Umfangswand des Behälters eine Zulaufanschlussbohrung 8 und eine Rücklaufanschlussbohrung 9 angeordnet sind. Zusätzlich zeigt die Fig. 7 zwei seitliche Spannringe 15, die um den dosenförmigen Behälter und den Deckel 3 in Pfeilrichtung aufgeschoben werden, um die Einheit aus Behälter und Deckel 3 durch eine nicht dargestellte Dreh- oder Rolleinrichtung um die Querachse in Pfeilrichtung 16 zu drehen oder zu rollen.

Fig. 8 zeigt wie in dem Inneren des Behälters ein eigener Zellkulturraum 6 gebildet ist. Anstelle eines Zellkulturraumes 6 kann auch eine Struktur vorgesehen sein, auf der die Zellen 7 gezüchtet werden. Der separate Zellkulturraum 6 oder die Struktur wird in diesem Falle über eine Druckbeaufschlagungseinrichtung 17 in Form einer Zylinder-/Kolbeneinheit mit Druck beaufschlagt.

In einem Kolbenraum 18 der Zylinder-/Kolbeneinheit 17 mündet die Zulaufanschlussbohrung 8, welche im Eingangsbereich durch ein Rückschlagventil 19 absperrbar ist. Durch einen Kolben 20 der Zylinder-/Kolbeneinheit 17 wird das über die Zulaufanschlussbohrung 8 eingebrachte Nährmedium unter Druck gesetzt, wobei sich dieser Druck bis in das Innere des Behälters fortsetzt. Über eine Rücklaufanschlussbohrung 9 auf der dem Zulauf gegenüberliegenden Seite des Behälters erfolgt eine Abführung von Nährmedium. Wenn man den Innenraum des Behälters entsprechend unter einen Überdruck setzen möchte, der gegebenenfalls wechselnd sein kann, wird man in diesem Falle den Rücklauf von Nährmedium drosseln oder die Rücklaufanschlussbohrung 9 entsprechend absperren.

Anstelle einer Zufuhr von Nährmedium über die Zulaufanschlussbohrung 8 kann hierfür auch eine gesonderte Bohrung in einen der beiden Deckel 3 oder 12 oder in der Umfangswand des Behälters vorgesehen sein. In diesem Falle kann man auch durch Gas, z.B. Luft, den Innenraum des Behälters und damit den Zellkulturraum 6 entsprechend unter Druck setzen.

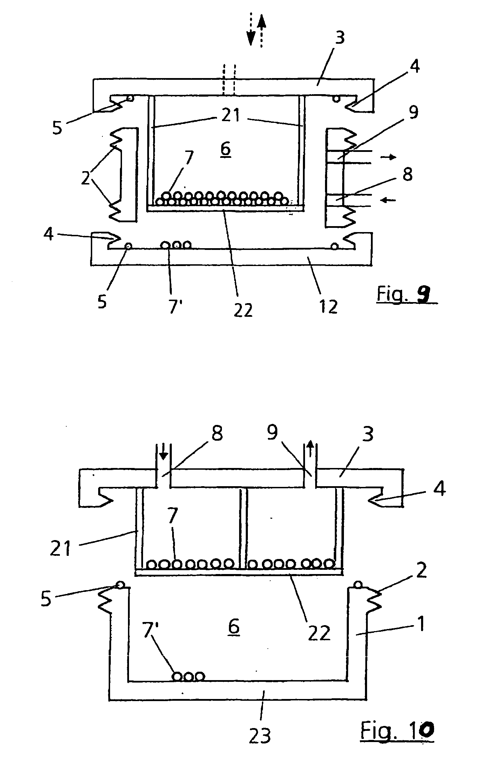

Die Fig. 9 zeigt eine Ausführungsform mit einem oberen Deckel 3 und einem unteren Deckel 12 und einem zylinderringförmigen Mittelteil eines Behälters. In diesem Falle ist der obere Deckel 3 mit einer Hängeeinrichtung in Form von mehreren über den Umfang verteilt angeordneten Stäben 21 versehen, die sich parallel zur Längsachse des Behälters in das Innere des Behälters erstrecken. Am unteren Ende der Stäbe 21 ist eine Plattform 22 befestigt, auf der die zu kultivierenden oder zu züchtenden Zellen 7 angeordnet werden. Die Zulaufanschlussbohrung 8 und die Rücklaufanschlussbohrung 9 können jeweils in der Umfangswand des Behälters angeordnet sein. Selbstverständlich ist jedoch auch eine Anordnung in einem der beiden Deckel 3 oder 12 möglich, wie dies gestrichelt angedeutet ist. Auch hier sind selbstverständlich auch getrennte Anschlussbohrungen für Zulauf und Ablauf möglich.

Der Vorteil der Ausführungsform mit der Hängeeinrichtung durch die Stäbe 21 besteht darin, dass sich auf diese Weise die Zellen 7 leichter in den Behälter einsetzen und daraus entnehmen lassen.

Die Verbindung der Stäbe 21 mit der Plattform 22 kann im Bedarfsfalle auch lösbar sein. Eine Lösbarkeit kann z.B. durch eine Clipverbindung erfolgen, wodurch ebenfalls eine leichtere Handhabung für die Vorrichtung erreicht wird.

Fig. 10 zeigt eine Ausführungsform, die ähnlich der in der Fig. 9 dargestellten Ausführungsform ist. Wie ersichtlich, weist sie nur einen oberen Deckel 3 und einen Behälter mit einem Boden 23 auf, wie er auch bei den übrigen Ausführungsformen mit nur einem Deckel 3 vorhanden ist. Die Plattform 22 ist in diesem Falle ebenfalls über Stäbe 21 mit dem oberen Deckel 3 verbunden.

Ein weiterer Vorteil der Ausführungsformen nach den Fig. 9 und 10 besteht darin, dass auf dem Behälterboden 23 bzw. der Innenseite des Deckels 12 zusätzlich noch eine weitere Möglichkeit für eine Züchtung oder Kultivierung von Zellen 7 gegeben ist. Auf diese Weise wird ein Zweikammersystem für die Kultivierung von zwei Zellkulturen geschaffen.

Anstelle von Zulaufanschlussbohrungen 8 und Rücklaufanschlussbohrungen 9 für Nährmedium kann selbstverständlich auch Nährmedium dauerhaft in den Behälter eingebracht werden und die Zulaufanschlussbohrungen 8 und die Rücklaufanschlussbohrungen 9 können dann nur zur Sauerstoffversorgung dienen.

Alternativ dazu ist es auch möglich, jeweils gesonderte Anschlussbohrungen für Sauerstoff und Nährmedium vorzusehen.

Die Plattform 22 kann als feste Einheit ausgebildet sein. Alternativ dazu ist es auch möglich, hierfür eine Membrane, z.B. eine poröse Membrane, vorzusehen, die eine Durchströmung von Sauerstoff zulässt.

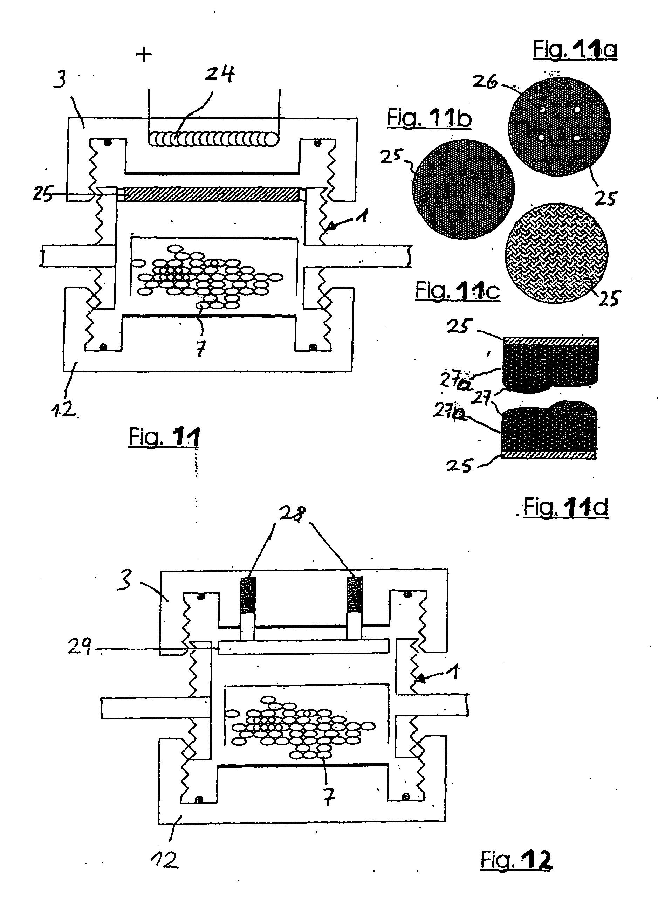

In den Figuren 11 bis 14 sind weitere Ausgestaltungen der Erfindung beschrieben, wobei der grundsätzliche Aufbau der Vorrichtung mit dem Behälter und den beiden Deckeln 3, 12 und/oder dem Boden 23 beibehalten worden ist, weshalb zur Vereinfachung nur die wesentlichen Bezugszeichen in den nachfolgend beschriebenen Figuren übernommen worden sind.

Fig. 11 zeigt einen Behälter, in welchem im Bereich des oberen Deckels 3 eine Magneteinrichtung 24, z.B. eine Magnetspule, die von Strom durchflössen wird, eingebaut ist.

Unter der Magneteinrichtung 24 befindet sich eine magnetisierbare Druckscheibe 25, welche in nicht näher dargestellter Weise elastisch mit dem Behälter verbunden ist.

Durch eine Bewegung der Druckscheibe 25 aufgrund Aktivierung der Magneteinrichtung 24, die z.B. durch wechselnde Stromrichtungen erzeugt wird, wird eine innere Druckbelastung auf die Zellen 7 ausgeübt.

Die Fig. 11a zeigt eine Draufsicht auf das Profil einer Druckscheibe 25, wobei kleine Öffnungen 26 vorgesehen sind, damit Kulturmedium, das sich im Inneren des Behälters befindet, passieren kann.

Die Fig. 11b und 11c zeigen alternative Druckscheiben 25 in Netzstruktur- oder Gitterstrukturform, damit Kulturmedium passieren kann.

Selbstverständlich kann die Magnetspule als Magneteinrichtung 24 auch außerhalb des Deckels 3 über diesem angeordnet werden. Bei dieser Ausgestaltung muss der Deckel 3 selbstverständlich aus nicht magnetisierbarem Material bestehen, z.B. Kunststoff. In diesem Fall kann man entsprechend große Magneteinrichtungen 24 vorsehen und damit entsprechend hohe Druckkräfte erzeugen.

Fig. 11d zeigt eine Ausgestaltung eines Implantates, wobei Knorpelprofile 27 auf einer mineralischen Matrix für Knochenersatz als Trägerstruktur 27a angeordnet sind. Dabei sind zwei übereinander angeordnete Trägerstrukturen vorgesehen, an welchen jeweils eine Druckscheibe 25 angeordnet ist. Die mineralische Matrix kann z.B. eine Knochenstruktur, z.B. aus Kalziumphosphat, sein.

Die mineralische Matrix kann auch andere Profile aufweisen, wie sie als Implantate benötigt werden, wie z.B. Gelenkstrukturen. Dabei kann selbstverständlich auch von der Kreisform abgewichen werden. Gleiches gilt grundsätzlich auch für den Behälter.

Fig. 12 zeigt eine Ausgestaltung mit expansionsfähigen Elementen 28, welche eine beweglich in dem Behälter oder dem Deckel 3 angeordnete Platte 29, ähnlich wie die Druckscheiben 25, axial verschieben und damit wechselnde Druckkräfte auf die Zellen 7 ausüben können. Als expansionsfähige Elemente können z.B. sogenannte Memory-Metalle oder Kunststoffe verwendet werden, die sich verformen und wieder in die alte Form zurückkehren. So gibt es z.B. auch Kunststoffe, die sich durch elektrische Veränderung ausdehnen können. Elemente mit Memoryfunktion reagieren z.B. auf bestimmte Temperaturen oder auf eine Ultraschall-Beschallung und ändern auf diese Weise ihre Lage, womit sie eine Bewegung der Platte 30 erzeugen. Auch Federeinrichtungen sind möglich, ebenso wie Motoren mit Akkumulatoren oder Batterien.

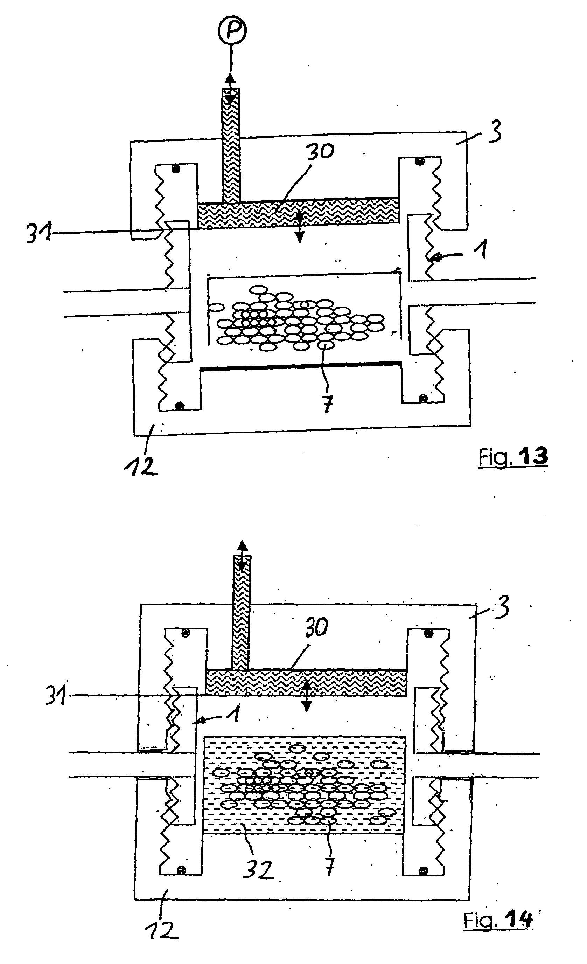

Fig. 13 zeigt ebenfalls eine innere Druckbeaufschlagung von Zellen 7 durch eine hydraulische oder pneumatische Einrichtung 30, die in dem Behälter bzw. in dem Deckel 3 angeordnet ist. Wie ersichtlich, weist die Einrichtung 30 eine bewegliche Folie, Platte oder Membrane 31 auf, hinter der sich eine hydraulische Flüssigkeit oder ein Gas als Fluid befindet. Die hydraulische Flüssigkeit oder das gasförmige Medium wird durch eine nicht näher dargestellte Druckeinrichtung P mit wechselndem Druck beaufschlagt, womit wechselnde Druckbelastungen auf die Zellen 7 ausgeübt werden. Anstelle einer elastischen Platte oder Membrane 31 kann im Bedarfsfalle auch ein Ballon verwendet werden, um eine größere Variationsmöglichkeit zu ergeben. Dabei können z.B. alle Wände des Behälters auf der Innenseite von einem derartigen Beutel oder Ballon umschlossen werden, wobei sich das Implantat bzw. die Zellkulturen im Inneren befinden. Auf diese Weise wird von außen her allseitig eine wechselnde Druckbelastung ausgeübt.

Die Fig. 14 zeigt eine ähnliche Ausgestaltung, wobei die Zellen 7 in einem Gel 32 liegen.

Im besonderen betrifft die Erfindung:
- Eine Vorrichtung zum Züchten oder Kultivieren von Zellen (7) bestehend aus einem dosenartigen Behälter, der aus einem oberen Deckel (3) und einem unteren als Boden dienenden Deckel (12) sowie einem zylinderförmigen Mittelteil (1) gebildet wird, welcher an beiden Stirnseiten druckdicht mit besagten Deckeln verbunden wird, wobei der Behälter mit wenigstens einer Anschlussbohrung (8), (9) zur Zufuhr und oder Abfuhr von Nährmedium versehen ist, und besagte Deckel (3, 12) und der zylinderförmige Mittelteil (1) durch eine Gewindeverbindung (2, 4) miteinander verbunden sind, wobei beide Deckel (3, 12) jeweils mit einem Verlängerungsring (14) versehen sind, welche wenigstens teilweise das zylinderförmige Mitteilteil (1) außenseitig dichtend umschließen, und dass weiterhin in dem Behälter eine Vorrichtung zur inneren Druckbeaufschlagung der Zellen (7) vorgesehen ist, wobei diese Druckbeaufschlagungsvorrichtung ausgewählt ist aus der Gruppe bestehend aus:
   (i) einer magnetisierbaren Druckscheibe (25), welche durch eine Magnetisierungseinrichtung (24) bewegbar ist;
   (ii) expansionsfähigen Elementen (28), welche eine beweglich angeordnete Platte (29) axial verschieben; und
   (iii) einer hydraulischen oder pneumatischen Einrichtung (30) mit einer beweglichen Folie, Platte, oder Membran (31).
- Eine entsprechende Vorrichtung, bei welcher die Druckscheibe (25) mit Bohrungen (26) versehen ist oder eine Gitter- oder Netzstruktur aufweist.
- Eine entsprechende Vorrichtung, bei welcher die Zellen (9) auf einer Trägerstruktur (27a) angeordnet sind, welche auf ein oder beiden Seiten mit einer Druckscheibe (25) versehen ist.
- Eine entsprechende Vorrichtung, bei welcher die expansionsfähigen Elemente (28) Memory-Metalle oder Memory-Kunststoffe sind, welche sich verformen und wieder in die alte Form zurückkehren können.
- Eine entsprechende Vorrichtung, bei welcher sich hinter der beweglichen Folie, Platte, oder Membran (31) eine hydraulische Flüssigkeit oder ein Gas-Fluid befindet, welche durch eine Druckeinrichtung mit wechselndem Druck beaufschlagt werden können.
- Eine entsprechende Vorrichtung, bei welcher Vorrichtung, bei welcher der Behälter auf beiden Seiten mit einem Spannring (15) zur Einleitung von Roll- oder Drehbewegung für den Behälter versehen ist.
- Eine Verwendung einer Vorrichtung wie oben und in den Ansprüchen dargelegt für die Züchtung und Kultivierung von Zellen unter in vivo Bedingungen und Sterilität im Massenbetrieb in einem aktiven Prozess bei kontinuierlicher oder diskontinuierlicher Zuführung von Nährmedium und Aussetzen der sich bildenden Zellkulturen mit Druck.

## Patentansprüche

1. Vorrichtung zum Züchten oder Kultivieren von Zellen (7) bestehend aus einem dosenartigen Behälter, der aus einem oberen Deckel (3) und einem unteren als Boden dienendenDeckel (12) sowie einem zylinderförmigen Mittelteil (1) gebildet wird, welcher an beiden Stirnseiten druckdicht mit besagten Deckeln verbunden wird, wobei der Behälter mit wenigstens einer Anschlussbohrung (8), (9) zur Zufuhr und oder Abfuhr von Nährmedium versehen ist, **dadurch gekennzeichnet, dass** besagte Deckel (3, 12) und der zylinderförmige Mittelteil (1) durch eine Gewindeverbindung (2, 4) miteinander verbunden sind, wobei beide Deckel (3, 12) jeweils mit einem Verlängerungsring (14) versehen sind, welche wenigstens teilweise das zylinderförmige Mitteilteil (1) außenseitig dichtend umschließen, und dass weiterhin in dem Behälter eine Vorrichtung zur inneren Druckbeaufschlagung der Zellen (7) vorgesehen ist, wobei diese Druckbeaufschlagungsvorrichtung ausgewählt ist aus der Gruppe bestehend aus:
(i) einer magnetisierbaren Druckscheibe (25), welche durch eine Magnetisierungseinrichtung (24) bewegbar ist;
(ii) expansionsfähigen Elementen (28), welche eine beweglich angeordnete Platte (29) axial verschieben; und
(iii) einer hydraulischen oder pneumatischen Einrichtung (30) mit einer beweglichen Folie, Platte, oder Membran (31).

2. Vorrichtung nach Anspruch 1, **dadurch gekennzeichnet, dass** die Druckscheibe (25) mit Bohrungen (26) versehen ist.

3. Vorrichtung nach Anspruch 1, **dadurch gekennzeichnet, dass** die Druckscheibe (25) eine Gitter- oder Netzstruktur aufweist.

4. Vorrichtung nach Anspruch 1, **dadurch gekennzeichnet, dass** die Zellen (9) auf einer Trägerstruktur (27a) angeordnet sind, welche auf ein oder beiden Seiten mit einer Druckscheibe (25) versehen ist.

5. Vorrichtung nach Anspruch 1, **dadurch gekennzeichnet, dass** die expansionsfähigen Elemente (28) Memory-Metalle oder Memory-Kunststoffe sind, welche sich verformen und wieder in die alte Form zurückkehren können.

6. Vorrichtung nach Anspruch 1, **dadurch gekennzeichnet, dass** sich hinter der beweglichen Folie, Platte, oder Membran (31) eine hydraulische Flüssigkeit oder ein Gas-Fluid befindet, welche durch eine Druckeinrichtung mit wechselndem Druck beaufschlagt werden können.

7. Vorrichtung nach einem der Ansprüche 1 - 6, **dadurch gekennzeichnet, dass** die Deckel (3, 12) ein Innengewinde und das zylinderförmige Mittelteil (1) ein Außengewinde besitzen.

8. Vorrichtung nach einem der Ansprüche 1 - 7, **dadurch gekennzeichnet, dass** die Gewindeverbindung mit wenigstens einem Dichtring (5) versehen ist.

9. Vorrichtung nach einem der Ansprüche 1 - 8, **dadurch gekennzeichnet, dass** der Deckel (3) mit einer Hängeeinrichtung (21) versehen ist, an der eine Plattform (22) zur Aufnahme der Zellen (7) angeordnet ist.

10. Vorrichtung nach einem der Ansprüche 1-9, **dadurch gekennzeichnet, dass** die Zellen (7) in einem Gel (32) angeordnet sind.

11. Vorrichtung nach einem der Ansprüche 1 - 10, **dadurch gekennzeichnet, dass** der Behälter auf beiden Seiten mit einem Spannring (15) zur Einleitung von Roll- oder Drehbewegung für den Behälter versehen ist.

12. Verwendung einer Vorrichtung gemäß der Ansprüche 1 - 11 für die Züchtung und Kultivierung von Zellen unter in vivo Bedingungen und Sterilität im Massenbetrieb in einem aktiven Prozess bei kontinuierlicher oder diskontinuierlicher Zuführung von Nährmedium und Aussetzen der sich bildenden Zellkulturen mit Druck.

## Claims

1. Apparatus for the culturing or cultivation of cells (7) consisting of a can-like container, which is formed from an upper lid (3) and a lower lid (12) serving as base, and a cylindrical central part (1), which is connected to said lids at both end faces in a pressure-tight manner, where the container is provided with at least one connection hole (8), (9) for the supply and/or discharge of nutrient medium, **characterized in that** said lids (3, 12) and the cylindrical central part (1) are connected to one another by means of a threaded connection (2, 4), where both lids (3, 12) are each provided with an extension ring (14), which at least partly surrounds the cylindrical central part (1) on the outside in a sealing manner, and **in that**, furthermore, a device for the internal pressurization of the cells (7) is provided in the container, where this pressurization device is selected from the group consisting of:
(i) a magnetizable pressure disc (25), which can be moved by a magnetization device (24);
(ii) expandable elements (28), which move a movably arranged plate (29) axially; and
(iii) a hydraulic or pneumatic device (30) having a movable film, plate or membrane (31).

2. Apparatus according to Claim 1, **characterized in that** the pressure disc (25) is provided with holes (26).

3. Apparatus according to Claim 1, **characterized in that** the pressure disc (25) has a grid or mesh structure.

4. Apparatus according to Claim 1, **characterized in that** the cells (9) are arranged on a support structure (27a), which is provided with a pressure disc (25) on one or both sides.

5. Apparatus according to Claim 1, **characterized in that** the expandable elements (28) are memory metals or memory plastics, which are able to deform and return to the original shape again.

6. Apparatus according to Claim 1, **characterized in that** a hydraulic fluid or a gas fluid, which can be subjected to varying pressure by a pressure device, is located behind the movable film, plate or membrane (31).

7. Apparatus according to one of Claims 1 - 6, **characterized in that** the lids (3, 12) have an internal thread and the cylindrical central part (1) has an external thread.

8. Apparatus according to one of Claims 1 - 7, **characterized in that** the threaded connection is provided with at least one sealing ring (5).

9. Apparatus according to one of Claims 1 - 8, **characterized in that** the lid (3) is provided with a suspension device (21), on which a platform (22) for the accommodation of the cells (7) is arranged.

10. Apparatus according to one of Claims 1 - 9, **characterized in that** the cells (7) are arranged in a gel (32).

11. Apparatus according to one of Claims 1 - 10, **characterized in that** the container is provided on both sides with a clamping ring (15) for the introduction of rolling or rotary motion for the container.

12. Use of an apparatus according to Claims 1 - 11 for the culturing and cultivation of cells under in-vivo conditions and sterility in mass operation in an active process with continuous or discontinuous supply of nutrient medium and pressurization of the cell cultures forming.

## Revendications

1. Appareil pour la culture ou la mise en culture de cellules (7), constitué par un conteneur en forme de boîte qui est formé à partir d'un couvercle supérieur (3) et d'un couvercle inférieur (12) jouant le rôle de base et à partir d'une partie centrale cylindrique (1), laquelle est connectée auxdits couvercles au niveau des deux faces d'extrémité d'une manière étanche à la pression, où le conteneur est muni d'au moins un trou de connexion (8), (9) pour l'application et/ou la décharge d'un milieu de nutriments, **caractérisé en ce que** lesdits couvercles (3, 12) et la partie centrale cylindrique (1) sont connectés les uns aux autres au moyen d'une connexion filetée (2, 4), où les deux couvercles (3, 12) sont chacun munis d'une bague d'extension (14) qui entoure au moins partiellement la partie centrale cylindrique (1) sur l'extérieur d'une manière étanche, et **en ce qu'**en outre un dispositif pour la pressurisation interne des cellules (7) est prévu dans le conteneur, où ce dispositif de pressurisation est choisi parmi le groupe constitué de :
(i) un disque de pression magnétisable (25), qui peut être déplacé par un dispositif de magnétisation (24) ;
(ii) des éléments extensibles (28), qui déplacent axialement une plaque agencée de façon mobile (29) ; et
(iii) un dispositif hydraulique ou pneumatique (30) comprenant un film, une plaque ou une membrane déplaçable (31).

2. Appareil selon la revendication 1, **caractérisé en ce que** le disque de pression (25) est muni de trous (26).

3. Appareil selon la revendication 1, **caractérisé en ce que** le disque de pression (25) a une structure de grille ou de maillage.

4. Appareil selon la revendication 1, **caractérisé en ce que** les cellules (9) sont agencées sur une structure de support (27a), qui est munie d'un disque de pression (25) sur l'un de ses côtés ou les deux.

5. Appareil selon la revendication 1, **caractérisé en ce que** les éléments extensibles (28) sont en métaux à mémoire de forme ou en plastiques à mémoire de forme, lesquels peuvent se déformer et revenir à nouveau à leur forme d'origine.

6. Appareil selon la revendication 1, **caractérisé en ce qu'**un fluide hydraulique ou un fluide gazeux, qui peut être soumis à une pression variable par un dispositif de pression, est situé derrière le film, la plaque ou la membrane déplaçable (31).

7. Appareil selon l'une des revendications 1 - 6, **caractérisé en ce que** les couvercles (3, 12) comportent un filet interne et la partie centrale cylindrique (1) comporte un filet externe.

8. Appareil selon l'une des revendications 1 - 7, **caractérisé en ce que** le joint fileté est muni d'au moins une bague d'étanchéité (5).

9. Appareil selon l'une des revendications 1 - 8, **caractérisé en ce que** le couvercle (3) est muni d'un dispositif de suspension (21), sur lequel une plate-forme (22) pour le logement des cellules (7) est agencée.

10. Appareil selon l'une des revendications 1 - 9, **caractérisé en ce que** les cellules (7) sont agencées dans un gel (32).

11. Appareil selon l'une des revendications 1 - 10, **caractérisé en ce que** le conteneur est muni sur ses deux côtés d'une bague de fixation (15) pour l'introduction au conteneur d'un mouvement de roulement ou de rotation.

12. Utilisation d'un appareil selon les revendications 1 - 11 pour la culture ou la mise en culture de cellules sous des conditions in vivo et sous un fonctionnement de stérilité en masse dans un processus actif avec une application continue ou discontinue d'un milieu de nutriments et une pressurisation de la formation des cultures de cellules.
